# EUROPEAN PATENT APPLICATION

(11) **EP 2 705 815 A1**
(43) Date of publication of application: **12.03.2014**
(21) Application number: 12183530.0
(22) Date of filing: 07.09.2012
(51) Int. Cl.: A61F 15/00

(54) **Device for use in a process of applying a bandage around a limb**

(71) Applicant: Dona +, 5504 KA Veldhoven (NL); Settels Savenije van Amelsvoort group of companies B.V., 5628 WB Eindhoven (NL); P&H Adviseurs Bouw-en Vastgoed B.V., 5504 KA Veldhoven (NL)
(72) Inventor: Dieleman, Maria, 6417 GR Heerlen (NL); van Rens, Piet Christiaan Jozef, 5721 LE Asten (NL); Dona, Marinus Josephus Jacobus, 5504 KA Veldhoven (NL); Peeters, Eugène Adrianus Franciscus Maria, 5504 KA Veldhoven (NL)
(74) Representative: Nederlandsch Octrooibureau

(57) **Abstract**

A handheld device (1) for use in a process of applying a bandage around a limb comprises a rotatable support (34) for supporting a bandage roll (40); a motor (31) for driving the support (34); a drive circuit (50) for driving the motor (31); an interface (60, 61) for receiving input information representing a desired tension of the bandage during a bandage applying process; and a controller (51) for controlling operation of the motor (31) during a bandage applying process through the drive circuit (50), which is connected to the interface (60, 61) for receiving the input information from the interface (60, 61), which is configured to determine a value representing an output torque of the motor (31) on the basis of the input information, and which is connected to the drive circuit (50) for setting the value in the drive circuit (50). The handheld device having a grip (10) for allowing a user of the device to take hold of the device, wherein the device further comprises a plate (2) for supporting both the grip (10) and the support (34).

## Description

The present invention relates to a device for use in a process of applying a bandage around a limb, comprising a rotatable support for supporting a bandage roll; and an interface for receiving input information representing a desired tension of the bandage during a bandage applying process.

For various medical reasons, it may be necessary to apply a bandage around a limb of a person or an animal in order to exert a certain pressure on the limb. For example, when a person is suffering from fluid-filled areas in the leg, a doctor may determine that the swollen leg is subjected to compression bandaging every day or every other day, so that it is eventually possible to use an elastic support stocking on the leg and to further treat the leg by means of the stocking. It is very difficult for medical staff members to perform the process of applying a bandage in such a way that a predetermined pressure is actually realized, as it is only possible for them to rely on feeling based on experience. As a result, in many practical cases, it appears that the pressure is too high or too low. Hence, there is a need for a device for use in a process of applying a bandage around a limb, which will hereinafter be referred to as bandage applicator, and which can assist the medical staff members with unwinding a bandage roll and wrapping the bandage around a limb in such a way that a correct pressure is obtained.

Various types of bandage applicators are known in the art. For example, WO 2011/025813 discloses a bandage applicator comprising a handle to be held by a user of the applicator, wherein the handle is coupled to a support in the form of a spindle. Furthermore, the applicator comprises a brake mechanism which is integral with the handle for controlling a tension of the bandage as it is applied to a limb. The bandage may be pre-wound on a spool that rotates around the spindle. Actuation of the braking mechanism increases pressure on the rotating spool in order to increase tension on the bandage. Alternatively, the spindle rotates in conjunction with a shaft within the handle. In that case, the braking mechanism applies pressure against the rotating shaft to increase tension in the bandage. Pre-wound cartridges of rolled bandage material may be successively loaded to the applicator's spindle for repeated usage of the applicator.

US 5,065,865 discloses a holder for a bandage and a tension roller journalled in the holder. The roller is controlled so that the force required to roll it is adjustable. The bandage is entrained over the roller and the surface of the roller is formed so that there is positive engagement of the bandage with the roller whereby the tension in the bandage is controlled. The holder includes an enclosure formed by end walls and curved piece, which serves to hold a bandage roll. From the enclosure, the bandage runs under an extension, thence over a drum or roller having an axle journalled in the side walls in front of the enclosure. This axle may take the form of a long bolt having a head at one end and a thumb nut threaded onto the bolt at the other. Bushings on the axle and between the ends of the roller and the side walls of the enclosure hold the rollers properly spaced, and provide braking for the roller as the thumb nut is tightened to press the side walls against the bushings. In order to have a possibility of replicating the pressure, a scale may be provided on the side wall under the nut. By noting the position of the nut on the scale when proper pressure is applied, and then using a similar setting of the nut, a duplicate -or near duplicate- of the pressure should be achieved.

GB 2 333 506 discloses a bandage applicator which comprises a support in the form of an axle around which a bandage having an adhesive element is wound. The axle is rotated within a handle, and a tension regulator is provided to control the degree of friction between the handle and the axle as the bandage is unwound from the applicator.

GB 2 369 815 discloses a bandage applicator which comprises a cylindrical body rotatable within a housing which forms a handle, and means at one end of the cylindrical body for mounting a bandage roll. The housing may be provided by two opposing half-casings, which are preferably cylindrical and concentric with the cylindrical body. The half-casings attach around a projection located on the cylindrical body, and further comprise interlocking studs and opposing grooves. The cylindrical body may also have a cylindrical extension fitted with longitudinal slots for engaging longitudinal ridges on a tubular body. The cylindrical body may have a projection in a region adjacent the handle, which, when pressurized by a user's finger, brakes the rotation of the cylindrical body within the housing in order to regulate the tension of the bandaging. The projection may further comprise linear ridges to facilitate the grip of the user's finger. The bandage material may be rolled around the tubular body, attached via an adhesive element. The bandage material may also have an adhesive element to allow the beginning of the bandage to be found.

It appears from the description of the various documents mentioned in the foregoing that it is known in the art to have a bandage applicator in which regulating the tension of the bandage involves applying pressure in order to have a braking action directly on the bandage, or on the support on which a bandage roll is mounted.

It is an objective of the present invention to provide another type of bandage applicator, particularly a bandage applicator in which the tension prevailing inside a bandage to be applied to a limb is set according to another principle than the known principle of performing a braking action in a mechanical manner, i.e. by having friction between at least two elements which are moving with respect to each other.

The objective of the present invention is achieved by means of a device for use in a process of applying a bandage around a limb, comprising a rotatable support for supporting a bandage roll; a motor for driving the support; a drive circuit for driving the motor; an interface for receiving input information representing a desired tension of the bandage during a bandage applying process; and a controller for controlling operation of the motor during a bandage applying process through the drive circuit, which is connected to the interface for receiving the input information from the interface, which is configured to determine a value representing an output torque of the motor on the basis of the input information, and which is connected to the drive circuit for setting the value in the drive circuit.

According to the present invention, controlling the tension in a bandage in a process of wrapping the bandage around a limb involves controlling an output torque of the motor which is connected to the support. Hence, realizing a correct tension is done in a most accurate way by setting an output torque instead of performing a braking action as known in the art. During operation of the bandage applicator according to the present invention, the motor acts to retract the bandage as long as a user of the bandage applicator exerts a pulling force on the bandage in a process of wrapping the bandage around a limb by means of the applicator. In case the user stops pulling at the bandage, for example, when putting the bandage applicator from one hand into another, the bandage tends to wind around the support again, as a result of which sagging of the bandage and loss of tension in the bandage are prevented. By controlling the output torque of the motor, it is guaranteed that the correct tension is always prevailing in the bandage.

The bandage applicator according to the present invention is very easy to use. In fact, a user of the bandage applicator does not need to work in another way as compared to a situation in which a bandage is applied around a limb by hand, i.e. without using a device. However, a major difference with such a situation is that the user is no longer required to rely on feeling when it comes to exerting a pulling force on the bandage while applying the bandage. According to the present invention, the user can simply pull at the bandage with a force which is sufficient for unwinding the bandage from the support, wherein the exact tension in the bandage is obtained automatically by appropriate control of the motor, particularly the output torque of the motor, which results in a certain pulling force through the bandage roll.

The exact value of the output torque is determined on the basis of input information. When the input information represents a higher tension of the bandage, the output torque is higher as well. The input information can be varied over time during a bandage applying process if so desired, for example, depending on the position of the bandage on a limb, wherein the output torque of the motor is varied in a similar manner.

In a practical embodiment of the bandage applicator according to the present invention, the motor is an electromotor, wherein the controller is configured to determine a value representing a supply of current to the motor on the basis of the input information, and to control the drive circuit for realizing the value. Hence, the working principle of this embodiment is based on the fact that the output torque of an electromotor is determined by the current that is supplied to the motor, wherein the current may be set. In this embodiment, the controller may be configured to determine a voltage to be set in the drive circuit. Setting a current on the basis of an input voltage is possible when the drive circuit is adapted to realize a direct relation between the voltage and the current. For example, this is the case when the drive circuit comprises a metal-oxide-semiconductor field-effect transistor (MOSFET), an operational amplifier and a resistor, wherein the operational amplifier is connected to the controller for receiving an incoming voltage set by the controller, and wherein the operational amplifier is arranged for letting a voltage across the resistor be the same as the incoming voltage and thereby determining a value of a current in the drive circuit and determining an opened/closed state of the MOSFET.

For realizing a predetermined tension in a bandage, the output torque of the motor is set at an appropriate value by setting the current to be supplied to the motor. As long as the current is kept at a constant value, the output torque is at a constant value, and the tension is at a constant value (not taking into account a small variation on the basis of the fact that the diameter of the bandage roll decreases during unwinding). By using a circuit as mentioned, in which a MOSFET, an operational amplifier and a resistor are arranged besides the motor and a power source, it is possible to link the value of the current directly to a value of an input voltage. The MOSFET is a type of transistor, which can be used as an electrically controlled switch. In this case, the operational amplifier is used for determining the opened/closed state of the MOSFET. The operational amplifier is an electrical component which can be used as a voltage follower, wherein a voltage drop across the resistor equals the input voltage at the operational amplifier. In this way, the input voltage can be used to directly determine the current to be supplied to the motor.

In respect of the power source, it is noted that it is advantageous to use a power source in the form of a battery. In such a case, optimal freedom of movement of the bandage applicator is obtained, which is important in the context of bandage applying processes.

In general, the bandage applicator according to the present invention can be a handheld device having a grip for allowing a user of the applicator to take hold of the device, wherein the device further comprises a plate for supporting both the grip and the support. The interface which is used for transferring input information to the controller of the bandage applicator can comprise a potentiometer, wherein a user can set the correct tension for a bandage by simply rotating a knob, for example.

The bandage applicator according to the present invention offers a possibility of receiving input information in an automated manner. In particular, the interface may be adapted to communicate with a computer through the Internet. Additionally or alternatively, the interface may be adapted to read information from a digital card.

On the other hand, it is possible for the bandage applicator to provide information about bandage applying processes which have been performed by means of the applicator. To that end, the bandage applicator may comprise a memory for storing information regarding the voltage in the drive circuit for driving the motor, which can be used as a representative of the tension in the bandage as explained in the foregoing. In general, the memory may be adapted to store information regarding the value representing the output torque of he motor as set in the drive circuit. In a practical embodiment, the bandage applicator further comprises a transmitter for transmitting information from the memory to a location outside of the applicator. In order to preserve a wireless appearance of the bandage applicator, the transmitter may be adapted to transmit information in a wireless fashion.

According to another option, the bandage applicator may be equipped with an encoder for registering length and position of applied bandages, wherein the encoder is located at an output shaft of the motor. Also, it is possible to provide a measuring device which is suitable to be put on the bandage applicator in the place of a bandage, i.e. on the support, and which is adapted to measuring dimensions of limbs, particularly a thickness of limbs. By performing measurements of the thickness of limbs, it is possible to check whether improvements are made by using the bandages and applying a certain pressure to the limbs by means of the bandages, and to which extent the thickness is decreased.

All in all, various ways of providing information to the bandage applicator and obtaining information from the bandage applicator are possible within the framework of the present invention. When the Internet is used, for example, it is possible for a doctor to monitor the bandage applying processes which are performed on a patient, and also to provide appropriate information for future processes. Especially when the electromotor as described in the foregoing and the electrical circuit for controlling the current to be supplied to the motor are used, it is relatively simple to equip the bandage applicator with suitable components for realizing exchange of information between the applicator and the external world.

In a further automated embodiment, the bandage applicator according to the present invention may comprise a scanner for reading information on a bandage roll representing the type of the bandage roll, wherein the scanner is connected to the controller. Various types of bandage rolls may be provided with various barcodes, and the scanner may be a barcode reader. It may be relevant to have information about the type of bandage roll, especially in view of the fact that rolls of self-adhesive bandage exist, which require a higher torque for unwinding.

The above-described and other aspects of the present invention will be apparent from and elucidated with reference to the following detailed description of a handheld bandage applicator which is adapted to set a required tension in a bandage during a bandage applying process by controlling an output torque of a motor which is present in the applicator for driving a support for supporting a bandage roll.

The present invention will now be explained in greater detail with reference to the figures, in which equal or similar parts are indicated by the same reference signs, and in which:
figure 1 shows a perspective view of the bandage applicator according to the present invention;
figure 2 shows a sectional view of the bandage applicator; and
figure 3 is an electrical diagram of a controller of the bandage applicator and a drive circuit for driving the motor of the bandage applicator.

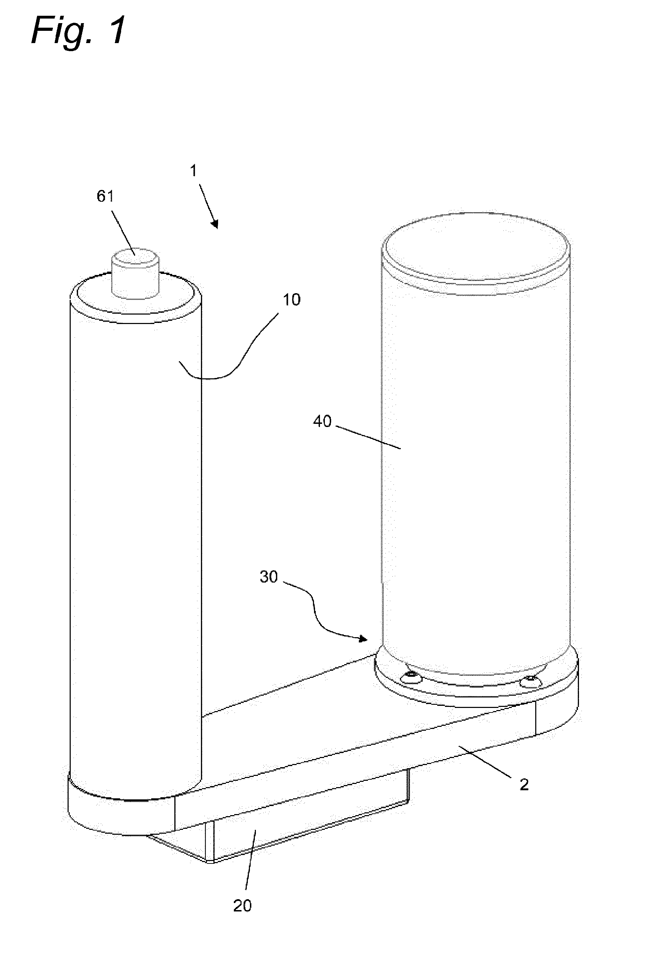

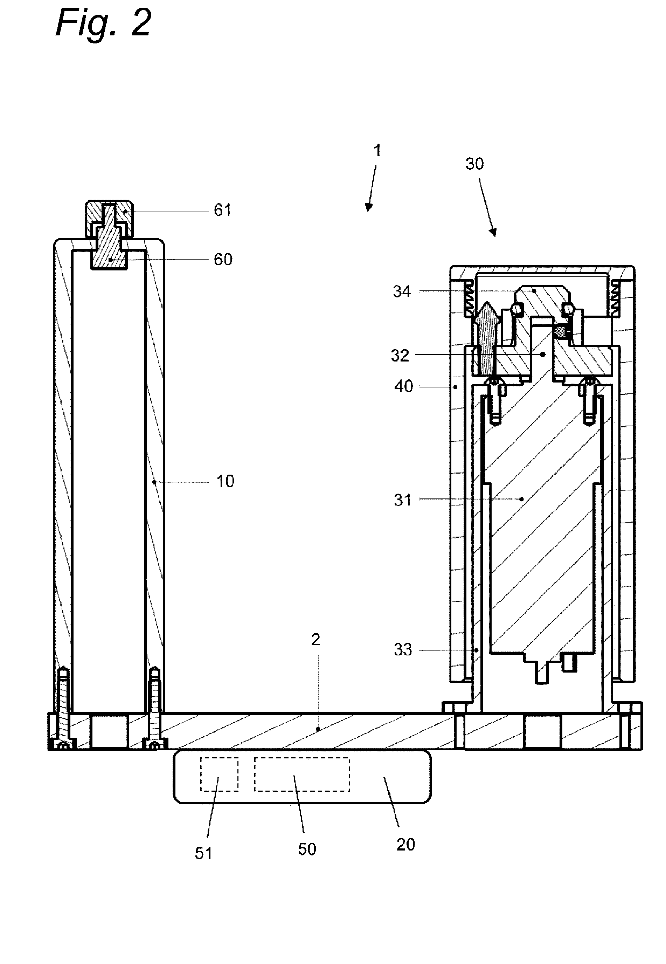

Figure 1 shows a perspective view of the bandage applicator 1 according to the present invention, and figure 2 shows a sectional view of the bandage applicator 1. The bandage applicator 1 is provided in the form of a handheld device, and comprises a grip 10 for allowing a user of the device to take hold of the device. The grip 10 is supported on a plate 2 which serves to support two other main components of the bandage applicator besides the grip 10, namely an electrical box 20 for accommodating a battery and various other electrical components as will be explained later on, and an assembly 30 for supporting and driving a bandage roll 40.

Figure 2 shows that the assembly 30 for supporting and driving a bandage roll 40 comprises an electromotor 31 having an output shaft 32, which is arranged in a housing 33, and further comprises a support 34 for supporting a bandage roll 40, wherein the support 34 is arranged on the output shaft 32 of the motor 31. Hence, the support 34 is driven by the motor 31 during operation of the bandage applicator 1. When it is intended to use the bandage applicator 1 in a process of applying a bandage around a limb such as a leg, a bandage roll 40 is provided and coupled to the support 34. The support 34 can be equipped with any suitable means for establishing a coupling which is both releasable and reliable.

The bandage applicator 1 offers a possibility for handling a bandage roll 40 and unwinding the bandage in a convenient manner, as a user of the applicator 1 can hold the grip 10 during a bandage applying process, and continuously move the applicator 1 such as to encircle to limb to be enwrapped in the bandage. Moreover, the bandage applicator 1 offers a possibility for realizing a correct tension in the bandage during the bandage applying process, as the applicator 1 is adapted to accurately control the operation of the motor 31 as will be explained in the following.

A principle underlying the design of the bandage applicator 1 is that the tension prevailing in a bandage is not controlled by braking the bandage, but by using the motor 31 for realizing a certain torque at the support 34. By using a motor 31 and controlling the output torque of the motor 31, the tension in the bandage is accurately set, independent of the extent to which a user of the bandage applicator 1 pulls at the bandage by means of the bandage applicator 1. In fact, the pulling action taken by a user causes the bandage to unwind from the roll 40 without influencing the tension prevailing in the bandage. If, during a bandage applying process, a user starts to pull harder at the bandage, the speed at which the bandage is unwound from the roll 40 is increased, while the tension in the bandage is not changed. If, during a bandage process, a user releases the bandage, the bandage is wound back on the roll 40 again until the user restores the pulling action.

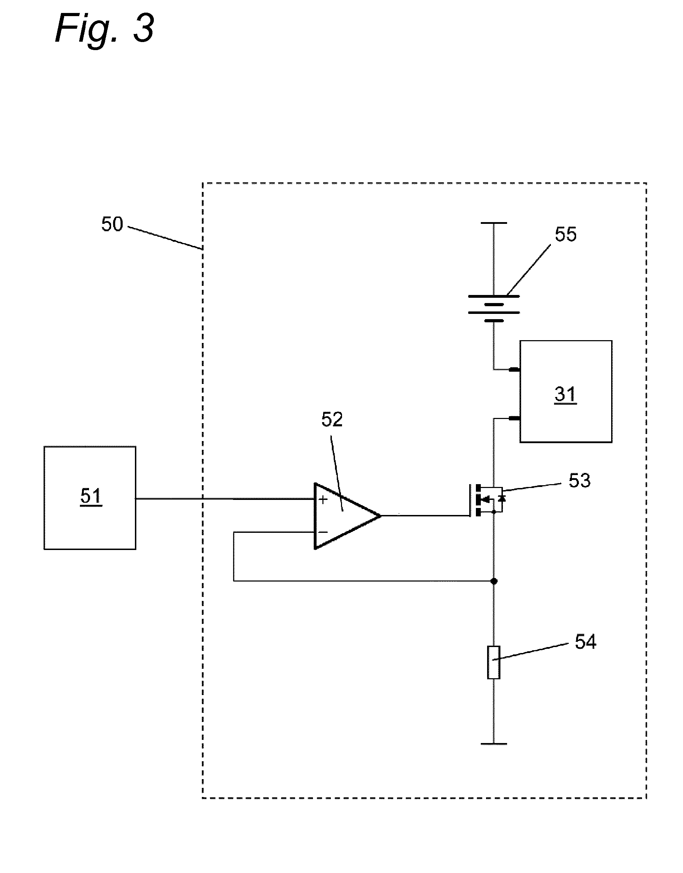

In general, for the purpose of controlling the operation of the motor 30 so that a desired tension is realized in a bandage, the bandage applicator 1 comprises a drive circuit for driving the motor 31, an interface for receiving input information representing the desired tension, and a controller which is connected to both the interface and the drive circuit. In the shown example, the drive circuit is a circuit 50 comprising electrical components as diagrammatically shown in figure 3, which are located inside the electrical box 20. The controller 51 can be a programmable chip, for example, and may be located inside the electrical box 20 as well. In figure 2, both the controller 51 and the drive circuit 50 are indicated by means of dashed boxes. In figure 3, the controller 51 is diagrammatically depicted in the form of a box which is connected to the drive circuit 50. Furthermore, in the shown example, the interface is in the form of a potentiometer 60 and an associated knob 61 by means of which a user of the bandage applicator 1 can adjust a setting of the potentiometer 60. For sake of completeness, it is noted that the bandage applicator 1 may be equipped with suitable wiring for connecting the potentiometer 60 to the controller 51.

The controller 51 is adapted to provide an input value to the drive circuit 50, particularly an input voltage which is determined by the controller 51 in relation to the setting of the potentiometer 60. The drive circuit 50 is designed to set an input current for the motor 31 on the basis of the input voltage, and to thereby determine an output torque of the motor 31 and an associated tension of the bandage. The input voltage is received by the drive circuit 50 at a side where a operational amplifier 52 is present. The drive circuit 50 further comprises a MOSFET 53, a resistor 54, and a battery 55. The battery 55 serves as a power source, whereas the value of the current at which the motor 31 is operated is determined by the operational amplifier 52, the MOSFET 53 and the resistor 54. In particular, the MOSFET 53 is put in an opened state when the value of the current is too low, and the MOSFET 53 is put in a closed state when the value of the current is too high, wherein the operational amplifier 52 is applied for controlling the state of the MOSFET 53. The operational amplifier 52 is arranged in the circuit 50 in such a way as to be capable of serving as a voltage follower, wherein a voltage drop across the resistor 54 is always equal to the input voltage. On the basis of the well-known fact that the current is directly related to the voltage through a factor which is determined by the resistance of the resistor 54 (V = I * R, wherein V represents the voltage, I represents the current, and R represents the resistance), the input voltage directly results in a current for the motor 31, independent of a voltage prevailing across the motor 31.

When the current by means of which the motor 31 is operated is kept at a constant value, the output torque of the motor 31 is kept at a constant value, and so is the tension prevailing in the bandage as it is unwound from the roll 40, regardless of other factors including the pulling force exerted by a user. The user can simply use the knob 61 of the potentiometer 60 for setting a desired value of the tension, as the setting of the potentiometer 60 determines the input voltage for the drive circuit 50 and the current at which the electromotor 31 is operated, resulting in a certain output torque of the motor 31 and an associated force acting on the bandage.

If different areas of a limb which is to be enwrapped in a bandage require different tension settings, a user can simply realize the correct values by putting the knob 61 of the potentiometer 60 in appropriate positions during various stages of a bandage applying process.

It follows from the foregoing that by using the bandage applicator 1, it is possible to have accurate control of the tension in a bandage to be applied to a limb such as a leg. This means that treatments of the limb can be most effective, wherein risks that bandages are too loose or too tight are minimized, and wherein it is not necessary to rely on experience of a person who is involved in applying the bandage as far as a feeling of the correct tension in the bandage is concerned.

It will be clear to a person skilled in the art that the scope of the present invention is not limited to the examples discussed in the foregoing, but that several amendments and modifications thereof are possible without deviating from the scope of the present invention as defined in the attached claims. While the present invention has been illustrated and described in detail in the figures and the description, such illustration and description are to be considered illustrative or exemplary only, and not restrictive. The present invention is not limited to the disclosed embodiments.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope of the present invention.

## Claims

1. Device (1) for use in a process of applying a bandage around a limb, comprising
- a rotatable support (34) for supporting a bandage roll (40);
- a motor (31) for driving the support (34);
- a drive circuit (50) for driving the motor (31);
- an interface (60, 61) for receiving input information representing a desired tension of the bandage during a bandage applying process; and
- a controller (51) for controlling operation of the motor (31) during a bandage applying process through the drive circuit (50), which is connected to the interface (60, 61) for receiving the input information from the interface (60, 61), which is configured to determine a value representing an output torque of the motor (31) on the basis of the input information, and which is connected to the drive circuit (50) for setting the value in the drive circuit (50).

2. Device (1) according to claim 1, wherein the motor (31) is an electromotor, and wherein the controller (51) is configured to determine a value representing a supply of current to the motor (31) on the basis of the input information, and to control the drive circuit (50) for realizing the value.

3. Device (1) according to claim 2, wherein the controller (51) is configured to determine a voltage to be set in the drive circuit (50).

4. Device (1) according to claim 3, wherein the drive circuit (50) comprises a metal-oxide-semiconductor field-effect transistor (MOSFET) (53), an operational amplifier (52) and a resistor (54), wherein the operational amplifier (52) is connected to the controller (51) for receiving an incoming voltage set by the controller (51), and wherein the operational amplifier (52) is arranged for letting a voltage across the resistor (54) be the same as the incoming voltage and thereby determining a value of a current in the drive circuit (50) and determining an opened/closed state of the MOSFET (53).

5. Device (1) according to any of claims 2-4, wherein the drive circuit (50) comprises a power source (55) in the form of a battery.

6. Device (1) according to any of claims 1-5, being a handheld device having a grip (10) for allowing a user of the device to take hold of the device, wherein the device further comprises a plate (2) for supporting both the grip (10) and the support (34).

7. Device (1) according to any of claims 1-6, wherein the interface comprises a potentiometer (60).

8. Device (1) according to claims 1-7, wherein the interface is adapted to communicate with a computer through the Internet.

9. Device (1) according to claim 1-8, wherein the interface is adapted to read information from a digital card.

10. Device (1) according to any of claims 1-9, further comprising a memory for storing information regarding the value representing the output torque of the motor (31) as set in the drive circuit (50).

11. Device (1) according to claim 10, further comprising a transmitter for transmitting information from the memory to a location outside of the device.

12. Device (1) according to claim 11, wherein the transmitter is adapted to transmit information in a wireless fashion.

13. Device (1) according to any of claims 1-12, further comprising a scanner for reading information on a bandage roll (40) representing the type of the bandage roll (40), wherein the scanner is connected to the controller (51).
